# EUROPÄISCHE PATENTSCHRIFT

(11) **EP 1 053 026 B1**
(45) Veröffentlichungstag und Bekanntmachung des Hinweises auf die Patenterteilung: **30.07.2003**
(21) Anmeldenummer: 99904842.4
(22) Anmeldetag: 02.02.1999
(51) Int. Cl.: A61K 49/00

(54) **KONTRASMITTEL AUF BASIS POLYOXYETHYLEN-660-12-HYDROXYSTEARAT UND ANIONISCHE PHOSPHOLIPIDE**
CONTRAST MEDIUM BASED ON POLYOXETHYLENE-660-12-HYDROXYSTEARATE AND ANIONIC PHOSPHOLIPIDS
PRODUIT DE CONTRASTE A BASE DE POLYOXYETHYLENE-660-12-HYDROXYSTEARATE ET D'UN PHOSPHOLIPIDE ANIONIQUE

(30) Priorität: 07.02.1998 DE 19805012
(43) Veröffentlichungstag der Anmeldung: 22.11.2000
(73) Patentinhaber: Gieselmann, Thomas, 56288 Kastellaun (DE)
(72) Erfinder: Gieselmann, Thomas, 56288 Kastellaun (DE)
(74) Vertreter: Müller-Gerbes, Margot, Dipl.-Ing.
(86) Internationale Anmeldenummer: EP9900649
(87) Internationale Veröffentlichungsnummer: WO99039745

(56) Entgegenhaltungen:
- WO-A-94/09703
- WO-A-96/18420
- WO-A-96/26746
- WO-A-98/17324
- DE-A- 4 328 642
- DE-A- 4 406 474
- CORSWANT VON C ET AL: "TRIGLYCERIDE-BASED MICROEMULSION FOR INTRAVENOUS ADMINISTRATION OF SPARINGLY SOLUBLE SUBSTANCES" JOURNAL OF PHARMACEUTICAL SCIENCES, Bd. 87, Nr. 2, 1. Februar 1998, Seiten 200-208, XP000729421
- "Fine chemicals: Pharma Ingredients: excipients" BASF AG: PHARMA INGREDIENTS,1999, Seite 1 XP002105137 www.basf.de/basf/html/e/produkte/gebiete/m er/pharma/cremo1.htm
- COON J. S.: "Solutol HS15, nontoxic polyoxyethylene esters of 12-hydroxystearic acid reverses multidrug resistance" CANCER RESEARCH, Bd. 51, 1. Februar 1991, Seiten 897-902, XP002105170
- BUCKINGHAM, L. E. ET AL: "Comparison of Solutol HS15, cremophor EL and novel ethoxylated fatty acid surfactants as multidrug resistance modification agents" INT. J. CANCER, Bd. 62, 1995, Seiten 436-442, XP002105171

## Beschreibung

Die Erfindung bezieht sich auf ein intravenös verabreichbares (IV) Kontrastmittel zur Verwendung als Diagnostikum bei bildgebenden Verfahren sowie seine Herstellung.

In der Medizin hat die Ultraschalldiagnostik wegen ihrer komplikationslosen und einfachen Handhabung zwischenzeitlich eine sehr breite Anwendung gefunden. Das Prinzip der Ultraschalldiagnostik beruht darauf, daß die Schallwellen von Organen und deren Grenzen unterschiedlich reflektiert werden. Durch elektronische Verstärkung werden diese Echosignale sichtbar gemacht.

Im normalen 2D-Bild (B-Mode) ist die Darstellung des Blutflusses von Gefäßen und inneren Organen nicht möglich. Blut und andere Flüssigkeiten liefern nur dann einen Kontrast im Ultraschall, wenn Dichteunterschiede zur Umgebung bestehen.

In der Medizin werden als Kontrastmittel im Ultraschallbereich Luft bzw. andere Gase verwendet, weil der Impedanzsprung zwischen Gas und umgebendem Blut wesentlich größer ist als der zwischen Flüssigkeiten oder Festkörpern und Blut.

Bei Ultraschalluntersuchungen des Herzens oder von Gefäßen ist es möglich, die schwachen Reflektionen des Ultraschalls an den roten Blutkörperchen zu nutzen, indem man sich des Doppler-Phänomens bedient. Hiermit ist es möglich, die Blutströme auch ohne Zugabe eines Kontrastmittels sichtbar zu machen.

Um aber den. Blutfluß in tieferliegenden Gefäßen messen bzw. auch sehr niedrige Fließgeschwindigkeiten detektieren zu können, ist die Beimischung kleiner Gasbläschen in den Blutstrom von Vorteil, weil die dadurch hervorgerufene stärkere Reflektion eine bessere Diagnostik ermöglicht.

Die aus der Literatur bekannten lungengängigen Echokontrastmittel funktionieren auf der Basis von Fremdgasen. Es gibt bisher keine Aussagen darüber, ob diese Kontrastmittel auf der Basis von Luft gleichfalls lungengängig wären, siehe beispielsweise Echocardiography: A Jrnl. of CV Ultrasound & Allied Tech. Vol. 14, No. 5, 1997 S. 441-446 von Thomas R. Porter, M.D., Feng Xie, M.D. und Shouping Li, M.D. "Differences in Myocardial Contrast Produced with Transient Response Imaging When Using Intravenous Microbubbles Containing Gases of Different Molecular Weight" oder "Drugs of the Future 1995, 20 (12): 1224-1227 "Levovist ® " von Dr. Thomas Fritzsch und Dr. med. Reinhard Schlief oder Echocardiography: A Jrnl. of CV Ultrasound & Allied Techn., Vol. 14, No. 4, 1997, S. 337-343 von Sameh Mobarek, M.D:, Marc Kates, D.O., Maria Meza, M.D., Carlos Moreno, Susan Revall, Wayne Barbee, Ph.D. "Identification of Perfusion Abnormalties Using FS069, a Novel Contrast Agent, in Conscious Dogs" oder Acta Radiologica 38 (1997), Supplement 412, 101-112 von J.-M. Correas, O. Hélénon, L. Pourcelot und J.-F. Moreau "Ultrasound Contrast Agents"

Zwecks Herstellung und anschließender Stabilisierung von Luft- bzw. Gasbläschen sind aus der Literatur mehrere Methoden bekannt. Durch heftiges Schütteln bzw. Rühren von Lösungen, wie Kochsalz- oder Farbstofflösungen oder von vorher entnommenem Blut, lassen sich Mikrobläschen erzeugen. Dadurch wird zwar ein Kontrast im Ultraschall erreicht, allerdings verbunden mit dem Nachteil der schlechten Reproduzierbarkeit und stark schwankender Größe der Gasbläschen. Durch verbesserte Verfahren werden diese Nachteile zum Teil aufgehoben.

Zwecks Anhebung des Kontrastes finden in der Ultraschalldiagnostik Zubereitungen Anwendung, die, wie zum Beispiel agitierte Röntgenkontrastmittel, feinstverteilte Gasbläschen enthalten. In der Literatur werden Galaktosepartikel beschrieben, an denen Luft adsorbiert wird und die freigesetzt werden, sobald diese mit einem geeigneten Lösungsmittel in Kontakt treten.

In der DE 44 06 474 A1 ist ein Mittel zur Verwendung in der Ultraschalldiagnostik beschrieben, das Mikropartikel enthält, die mit vorwiegend lipophilen Stoffen überzogen sind und dadurch ein rasches Auflösen der Galaktose verhindern. Diese Mikropartikel werden in ein Behältnis gefüllt, mit einem gasförmigen, halogenierten Kohlenwasserstoff überschichtet und vor Gebrauch in Wasser suspendiert. Es liegt hier eine Suspension von Mikropartikeln in Wasser oder in einer wässrigen Lösung vor.

In der WO 93/13808 wird ein Kontrastmittel beschrieben, bei dem Mikropartikel, zum Beispiel Galaktosepartikel, mit einem Emulgator überzogen werden. Unmittelbar vor Anwendung werden die Partikel in einer wässrigen Lösung suspendiert. Es liegen hier ebenfalls Partikel in einer Suspension vor.

In der WO 96/26746 werden als Ultraschallkontrastmittel stabilisierte Zubereitungen beschrieben, die einen hydrophilen Stoff, zum Beispiel Hydroxyethylstärke, in Kombination mit einem neutralen Phospholipid (Phosphatidylcholin) und einen weiteren Emulgator enthalten. Nach Zugabe eines wässrigen Lösungsmittels und einem geeigneten Gas wird die Mischung appliziert. Es wird kein negativ geladenes (anionisches Phospholipid verwendet und es liegen anstatt einer Lösung Liposome (Partikel) vor. Die Liposome oder oberflächenaktive, visköse Lösungen oder eine Lösung gemäß der DE 43 28 642 A1 werden mit physiologisch verträglichen Gasen sonkiert und tiefgefroren gelagert.

Ebenfalls ist ein aufgeschäumtes und anschließend denaturiertes Protein beschrieben worden und auch eine Substanz, die ein Tensid bzw. ein Tensidgemisch und eine viskositätserhöhende Substanz enthält.

Aus den DE 196 26 530 A1, DE 43 28 642 A1, EP 0494 615 A1, WO 92/11873 sind wässrige Zubereitungen zur Verwendung als Echokontrastmittel bekannt, die von einer Mischung aus Polyoxyethylen-Polyoxypropylen-Polymer mit negativ geladenen Phospholipiden ausgehen, die klar gelöst sind.

Bei den augenblicklich zur Verfügung stehenden Kontrastmitteln spielen bezüglich eines eventuellen Risikos hauptsächlich zwei Faktoren eine übergeordnete Rolle: die Größe und die Anzahl sowohl der Gasbläschen als auch der Feststoffpartikel.

Der Erfindung liegt die Aufgabe zugrunde, ein Ultraschallkontrastmittel zu schaffen, das die nachfolgend geforderten Eigenschaften besitzt:
1. Lungengängigkeit
2. Kapillargängigkeit
3. Minimierung des Embolierisikos
   - Gasbläschen (Größe und Anzahl)
   - Feststoffpartikel (Größe und Anzahl)
4. Reproduzierbarkeit
5. Sterile und pyrogenfrei Zubereitung
6. Leichte Herstellung bei vertretbaren Kosten
7. Problemlose Bevorratung
8. Ausreichend lange Lagerstabilität
9. Wirtschaftlich in der Anwendung.

Diese Aufgabe wird erfindungsgemäß durch ein IV-Kontrastmittel gemäß Anspruch 1 und das Verfahren zu seiner Herstellung gemäß Anspruch 11 gelöst. Erfindungsgemäß wird als Kontrastmittel für intravenöse Verabreichung - IV - eine Zubereitung, enthaltend eine wässrige Lösung von Polyoxyethylen-660-12-Hydroxystearat und eines anionischen Phospholipides, eingesetzt.

Die Erfindung schlägt somit zur Lösung der gestellten Aufgabe eine klare, wässrige Lösung einer Mischung von zwei oberflächenaktiven Stoffen vor, die gegebenenfalls noch Stoffe zur Isotonisierung enthält und in die vor Gebrauch ein physiologisch verträgliches Gas eingearbeitet wird.

Diese erfindungsgemäße Zubereitung wird zum Kontrastmittel weitergebildet durch Einarbeitung eines physiologisch verträglichen Gases in Form feinstverteilter Gasbläschen. Man erhält ein Kontrastmittel, das aus Gasbläschen besteht, die ein Gas und Polyoxyethylen-660-12-Hydroxystearat und ein negativ geladenes Phospholipid in wässriger Lösung enthalten.

Die Erfindung ist ein Ultraschallkontrastmittel
- auf Basis von Luft oder anderer, physiologisch verträglicher Gase,
- das einen deutlichen Kontrast zum umliegenden Gewebe liefert,
- bei dem die Gasbläschen so klein und stabil sind, daß sie nach IV-Injektion sowohl qualitativ als auch quantitativ, verlustfrei das Linksherz erreichen,
- das sich, ohne allergenes Potential durch gute Verträglichkeit auszeichnet,
- bei dem die Gasbläschen weder in Blut noch in Wasser miteinander verklumpen,
- das sich einfach herstellen läßt.

Vorteilhafte Weiterbildungen und erfindungsgemäße Ausgestaltungen des IV-Kontrastmittels sind den kennzeichnenden Merkmalen der Ansprüche 2 bis 10 entnehmbar.

Die im Folgenden beschriebenen Zubereitungen unterscheiden sich vom augenblicklichen Stand der Technik dadurch, daß es sich hier um ein Echokontrastmittel handelt, das, entstanden durch geringen mechanischen Aufwand, Mikroluft- bzw. Mikrogasbläschen enthält und das wegen seiner großen Stabilität, lungengängig ist.

Die Erfindung schlägt zur Herstellung des IV-Kontrastmittels ein Verfahren gemäß Anspruch 11 vor. Vorteilhafte erfinderische Weiterbildungen des Verfahrens zum Herstellen des IV-Kontrastmittels sind den Merkmalen der Ansprüche 12 bis 16 entnehmbar.

Hierbei ist besonders hervorzuheben, daß sich das erfindungsgemäße Kontrastmittel durch Variation physiologisch verträglicher, einzuarbeitender Gase modulieren läßt. So ist es hierbei möglich, aus ein und derselben Zubereitung unter Verwendung körpertemperierter, gasförmiger Flurcarbone - vorwiegend C₃F₈ bis C₆F₁₄ - ein wesentlich langlebigeres Echokontrastmittel bzw. NMR-Kontrastmittel herzustellen. Unter Verwendung teilbromierter Fluorcarbone entsteht erfindungsgemäß ein als Röntgenkontrastmittel verwendbares Konstrastmittel.

Das für das Kontrastmittel benötigte Gas (Luft) wird der Zubereitung auf mechanischem Wege hinzugefügt. Das von den Gasbläschen transportierte Gasvolumen beträgt 0,01 bis 0,1 ml pro ml Zubereitung. Die Gasbläschen werden durch mechanische Zubereitung generiert und mit dem je nach Indikation zu verwendenden Gas beladen.

Durch Zugabe osmotisch aktiver Substanzen kann die physiologische Isotonie des Kontrastmittels hergestellt werden.

Bei dem oben beschriebenen Verfahren zur Herstellung des erfindungsgemäßen Kontrastmittels können Größenverteilungen der Gasbläschen erreicht werden, von denen 90 % kleiner als 3 µm sind. Pro Injektion werden 1 ml bis 10 ml des Kontrastmittels benötigt.

Erfindungsgemäß findet das IV-Kontrastmitel Verwendung als aufgeschäumte Zubereitung zur intravenösen Injizierung als Diagnostikum bei bildgebenden Verfahren.

Nutzt man das erfindungsgemäße IV-Kontrastmittel als Ultraschallkontrastmittel, ist nach IV-Applikation nicht nur die Darstellung des rechtsventrikulären Teils des Blutkreislaufs möglich.

Das Kontrastmittel kann vielmehr auch mit exzellenten Ergebnissen bei Untersuchungen des Linksherzes und des Myocards eingesetzt werden.

Gleichfalls können andere von Blut versorgte Organe, wie Leber, Milz, Niere und Gehirn, mit o.g. Kontrastmittel sichtbar gemacht werden.

Das erfindungsgemäße Kontrastmittel ist aber auch zum Sichtbarmachen von Hohlräumen in Mensch und Tier geeignet, wie zum Beispiel zur Darstellung der Harnblase, des Harnleiters, des Uterus oder der Vagina.

Überraschenderweise wurde gefunden, daß eine Lösung, bestehend aus Polyoxyethylen-660-12-Hydroxystearat und einem anionischen Phospholipid, in der Lage ist, durch Agitation eingearbeitete, physiologisch verträgliche Gase ebenfalls im arteriellen Kreislauf nach IV-Applikation die akustische Impedanz deutlich zu erhöhen.

In den nachfolgenden zwei Beispielen wird die Erfidnung detailliert beschrieben, wobei sich die Prozentangaben, soweit nicht anders angegeben, stets auf das Gewicht (g/v) beziehen.

Die wässrige Lösung enthält 0,5 bis 15 Gew.-% Polyoxyethylen-660-12-Hydroxystearat (bevorzugt 1 bis 5 Gew.-%) und 0,1 bis 10 Gew.-% anionischen Phospholipiden (bevorzugt 0,5 bis 2 Gew.-%). Der Lösung können auch noch physiologisch geeignete Stoffe zur Einstellung der Isotonie beigegeben werden.

| | | |
|---|---|---|
| Beispiel 1: | Polyoxyethylen-660-12-Hydroxystearat | 3,00 g |
| | Dimyristoylphosphatidylglycerol | 1,00 g |
| | Natriumchlorid | 0,90 g |
| | Aqua dest | auf 100,00 ml. |

Die Herstellung erfolgt, indem das Polyoxyethylen-660-12-Hydroxystearat bei ca. 65 bis 70°C geschmolzen wird. Anschließend löst man darin das anionische Phospholipid und gibt dann das Wasser und das Natriumchlorid unter Rühren langsam hinzu. Der pH-Wert wird mit verdünnter Natronlauge oder Salzsäure auf 7,0 bis 8,0 eingestellt.

| | | |
|---|---|---|
| Beispiel 2: | Polyoxyethylen-660-12-Hydroxystearat | 2,00 g |
| | Dipalmitooylphosphatdidylglyceral | 1,00 g |
| | Glycin | 1,50 g |
| | Aqua dest. | auf 100,00 ml. |

Das Polyoxyethylen-660-12-Hydroxystearat wird bei ca. 65 bis 70°C geschmolzen. Man löst darin das Phospholipid und gibt anschließend langsam das Wasser (65 bis 70°C) hinzu. Das Glycin wird gelöst. Mit Hilfe verdünnter Natronlauge bzw. Salzsäure wird ein pH von 7,0 bis 8,0 eingestellt.

Zwecks Erzeugung der Mikrogasbläschen kommen alle physiologisch verträglichen Gase in Frage. 1 ml der erfindungsgemäßen Zubereitung wird mit 0,01 bis 0,01 ml Gas aufgeschäumt. Die so entstandenen Mikrobläschen werden vorzugsweise IV injiziert. Ausgerichtet an der diagnostischen Fragestellung kommen 1 bis 20 ml der erfindungsgemäßen Zubereitung zur Injektion.

### Herstellung des gebrauchsfertigen Kontrastmittels

Die Gasbläschen sollten möglichst erst kurz vor der Anwendung am Patienten auf bereits bekannte Weise generiert werden. Bietet man o.a. Substanz beispielsweise in einer Durchstechflasche an, so kann die Lösung zusammen mit der benötigten Menge Luft bzw. Gas in eine marktübliche Spritze aufgezogen werden, die dann mit einer zweiten verbunden wird, um die Lösung unter Druck hin- und herpumpen zu können. Durch diesen Vorgang werden dann die Mikrobläschen generiert. Das Verbindungsstück zur zweiten Spritze kann hierbei ein 3-Wegehahn, ein Adapter mit Querschnittsverengung oder eine anderweitig, strömungsmechanisch wirksame Konstruktion sein.

### Echocardiographische Untersuchungen am Hund

Unter Zuhilfenahme zweier Spritzen, die durch einen 3-Wegehahn miteinander verbunden sind, wird die vorher aufgezogene, erfindungsgemäße Substanz hin- und hergepumpt. Die hierdurch entstandenen Gasbläschen werden in eine periphere Vene injiziert. Anschließend wird mit physiologischer Kochsalzlösung gespült. ein handelsüblicher für die Echocardiographie geeigneter Ultraschallkopf wird vor, während und nach der Injektion auf die für einen Längsschnitt durch das Links- und Rechtsherz typische Stelle aufgesetzt.

Unmittelbar nach der Injektion kann man nun auf dem Monitor des Ultraschallgerätes verfolgen, wie das durch das Kontrastmittel markierte Blut über den rechten Vorhof den rechten Ventrikel erreicht und diesen über die Lungenarterie wieder verläßt. Nach der Lungenpassage sind die Höhlen des Linksherz sehr gut erkennbar, während sich die des Rechtsherz wieder leeren. Hierbei ist festzustellen, daß das erfindungsgemäße Kontrastmittel im linken Herzen etwas länger verbleibt als im rechten. Die Intensität des Kontrastes im Ultraschallbild ist in beiden Herzhälften nahezu identisch, so daß davon auszugehen ist, daß die sich in den Gasbläschen befindliche Luft nicht wesentlich reduziert und somit ein nahezu verlustfreier Transport der Gasbläschen selbst durch die Lungenpassage zum linken Herzen gewährleistet ist.

Bei Wiederholung des Versuchs mit Gasbläschen, die mit Gas (vorzugsweise mit Fluorcarbonen) gefüllt sind, ist erstaunlicherweise festzustellen, daß sich die Kontrastdauer in beiden Herzhälften verlängert.

Als Resultat ergibt sich, daß die erfindungsgemäße Substanz, im Gegensatz zum augenblicklichen stand der Entwicklung in diesem Bereich, auf einfache Weise und reproduzierbar durch Austausch der Gase in seinen bildgebenden Eigenschaften nach Bedarf verändert werden kann. Hierdurch wird gleichzeitig die Palette der Möglichkeiten in der Ultraschalldiagnostik (Herz, Gefäße, Tumorperfusion etc.) erheblich erweitert.

Das erfindungsgemäße IV-Kontrastmittel zeichnet sich aus durch
- Verdünnbarkeit
- Infusionsfähigkeit
- vergleichbar mit Indikationen in der Radiologie
- für MRI einsetzbar
- Rezirkulation
Wegen seiner guten Verträglichkeit ist es auch möglich, das erfindungsgemäße IV-Kontrastmittel intraarteriell zu verabreichen.

Das in den Beispielen eingesetzte Polyethylenglycol-660-12-Hydroxystearat hat folgende Spezifikation:

| | |
|---|---|
| Erstarrungspunkt | 25-30°C |
| Verseifungszahl | 53-63 |
| Hydroxylzahl | 90-110 |
| Säurezahl | ≤ 1 |
| Wassergehalt (n.K.F) | ≤ 0,5 % |
| pH-Wert, 10 % in Wasser | 6-7 |
| Farbe 20 % in Wasser | nicht dunkler als Farblösung G 5 |
| Viskosität, 30 % in Wasser (25°C) | ca. 12 mPa-s |
| Sulfatasche | ≤ 0,3 % |
| Schwermetalle | ≤ 10 ppm |
| Ethylenoxid | ≤1 ppm |

Die Bestimmungsmethoden sind, soweit nicht anderes angegeben ist, dem derzeit gülten DAB/Ph.Eur. zu entnehmen.

Die Zusammensetzung besteht aus Polyglykolester der 12-Hydroxystearinsäure (70 %) = hydrophober Teil, Polyethylenglykol (30 %) = hydrophiler Teil.

12-Hydroxystearinsäure (12 HSA) ist die Hauptfettsäurekomponente, daneben sind noch Stearinsäure und Palmitinsäure nachweisbar.

## Patentansprüche

1. IV-Kontrastmittel, enthaltend eine Zubereitung auf Basis einer wässrigen Lösung von Polyoxyethylen-660-12-Hydroxystearat und eines anionischen Phospholipides.

2. IV-Kontrastmittel nach Anspruch 1,
**dadurch gekennzeichnet, daß** die Zubereitung auf Basis einer wässrigen Lösung von Polyoxyethylen-660-12-Hydroxystearat und eines anionischen Phospholipides durch Agitation eingearbeitete physiologisch verträgliche Gasen in Form feinstverteilter Gasbläschen enthält.

3. IV-Kontrastmittel nach Anspruch 1 oder 2,
**dadurch gekennzeichnet, daß** als physiologisch verträgliches Gas Luft verwendet ist.

4. IV-Kontrastmittel nach Anspruch 1 oder 2,
**dadurch gekennzeichnet, daß** als physiologisch verträgliches Gas gasförmige Fluorkohlenstoffe vorwiegend aus der Gruppe C₃F₈ bis C₆F₁₄ verwendet sind.

5. IV-Kontrastmittel nach einem der Ansprüche 1 oder 2,
**dadurch gekennzeichnet, daß** als physiologisch verträgliches Gas gasförmige teilbromierte Fluorkohlenstoffe verwendet sind.

6. IV-Kontrastmittel nach einem der Ansprüche 1 bis 5,
**dadurch gekennzeichnet, daß** 90 % der Gasbläschen einen mittleren Durchmesser kleiner 3 µm aufweisen.

7. IV-Kontrastmittel nach einem der Ansprüche 1 bis 6,
**dadurch gekennzeichnet,** eine osmotisch aktive Substanz, wie Natriumchlorid, Glycin, zur Herstellung der physiologischen Isotonie enthalten ist.

8. IV-Kontrastmittel nach einem der Ansprüche 1 bis 7,
**dadurch gekennzeichnet, daß** die wässrige Lösung
0,5 bis 15 Gew.-%, vorzugsweise 1 bis 5 Gew.-% Polyoxyethylen-660-12-Hydroxystearat und
0,1 bis 10 Gew.-%, bevorzugt 0,5 bis 2 Gew.-% Phospholipide enthält.

9. IV-Kontrastmittel nach einem der Ansprüche 1 bis 8,
**dadurch gekennzeichnet, daß** als Phospholipide Dimyristoylphosphatidylglycerol eingesetzt ist.

10. IV-Kontrastmittel nach einem der Ansprüche 1 bis 8,
**dadurch gekennzeichnet, daß** als Phospholipid ein Dipalmitoylphosphatidylglycerol eingesetzt ist.

11. Verfahren zum Herstellen eines TV-Kontrastmittels zur Verwendung als Diagnostikum bei bildgebenden Verfahren gemäß einem der Ansprüche 1 bis 10, **dadurch gekennzeichnet, daß** eine Zubereitung hergestellt wird, indem Polyoxyethylen-660-12-Hydroxystearat bei einer Temperatur von etwa 65 bis 75°C geschmolzen wird, danach ein anioniscches Phospholipid in dieser Schmelze gelöst wird und danach destilliertes Wasser und gegebenenfalls Natriumchlorid oder Glycin unter Rühren hinzugegeben wird.

12. Verfahren nach Anspruch 11,
**dadurch gekennzeichnet, daß** das destillierte Wasser in auf eine Temperatur von 65 bis 75°C vorgewärmtem Zustand zugegeben wird.

13. Verfahren nach Anspruch 11 oder 12,
**dadurch gekennzeichnet, daß** der pH-Wert der Zubereitung durch Zugabe von Natronlauge oder Salzsäure auf einen Wert von 7,0 bis 8,0 eingestellt wird.

14. Verfahren nach einem der Ansprüche 11 bis 13,
**dadurch gekennzeichnet, daß** der Zubereitung physiologisch verträgliches Gas im Volumenverhältnis Zubereitung zu Gas von 1 zu 0,1 bis 1 zu 0,01 zugegeben wird und durch Agitation (mechanisches Mischen) Gasbläschen erzeugt werden.

15. Verfahren nach Anspruch 14,
**dadurch gekennzeichnet, daß** Gasbläschen erzeugt werden, von denen mindestens 90 % einen mittleren Durchmesser kleiner als 3 µm aufweisen.

16. Verfahren nach einem der Ansprüche 11 bis 15,
**dadurch gekennzeichnet, daß** eine Zubereitung aus
0,5 bis 15 Gew.-Teilen Polyoxyethylen-660-12-Hydroxystearat und
1 bis 5 Gew.-Teilen eines Phospholipides und
100 Gew.-Teilen destilliertem Wasser und
0,5 bis 2 Gew.-Teilen einer osmotisch aktiven Substanz zum Einstellen des pH-Wertes, wie Natriumchlorid oder Glycin, hergestellt wird und
100 Volumenteile der Zubereitung mit 1 bis 10 Volumenteilen eines physiologisch verträglichen Gases
unter Ausbildung von Gasbläschen aufgeschäumt werden.

## Claims

1. An IV contrast medium, containing a preparation on the basis of an aqueous solution of polyoxyethylene-660-12-hydroxystereate and an anionic phospholipid.

2. The IV contrast medium in accordance with claim 1, **characterized in that** the preparation on the basis of an aqueous solution of polyoxyethylene-660-12-hydroxystereate and an anionic phospholipid contains physiologically compatible gases in the form of extremely finely distributed small gas bubbles introduced by means of agitation.

3. The IV contrast medium in accordance with claim 1 or 2, **characterized in that** air is used as the physiologically compatible gas.

4. The IV contrast medium in accordance with claim 1 or 2, **characterized in that** gaseous fluorocarbons, preferably from the group C₃F₈ to C₆F₁₄ are used as the physiologically compatible gas.

5. The IV contrast medium in accordance with one of claims 1 or 2, **characterized in that** gaseous, partially bromated fluorocarbons are used as the physiologically compatible gas.

6. The IV contrast medium in accordance with one of claims 1 to 5, **characterized in that** 90% of the small gas bubbles have an average diameter of less than 3µm.

7. The IV contrast medium in accordance with one of claims 1 to 6, **characterized in that** an osmotically active substance, such as sodium chloride, glycin, is contained for producing the physiological isotonicity.

8. The IV contrast medium in accordance with one of claims 1 to 7, **characterized in that** the aqueous solution contains
0.5 to 15 weight-%, preferably 1 to 5 weight-%, of polyoxyethylene-660-12-hydroxystereate and
0.1 to 10 weight-%, preferably 0.5 to 2 weight-%, of anionic phospholipids.

9. The IV contrast medium in accordance with one of claims 1 to 8, **characterized in that** dimyristoylphosphatidylglycerol is employed as the phospholipid.

10. The IV contrast medium in accordance with one of claims 1 to 8, **characterized in that** a dipalmitoylphosphatedidylglycerol is employed as the phospholipid.

11. A method for producing an IV contrast medium for use as a diagnostic agent in connection with image-producing methods in accordance with one of claims 1 to 10, **characterized in that** a preparation is made by melting polyoxyethylene-660-12-hydroxystereate at about 65 to 70°C, thereafter dissolving an anionic phospholipid in this molten mass and then adding distilled water and, if required, sodium chloride or glycin while stirring.

12. The method in accordance with claim 11,
**characterized in that** the distilled water is added in a state where it has been preheated to 65 to 75°C.

13. The method in accordance with claim 11 or 12,
**characterized in that** the pH value of the preparation is adjusted to 7.0 to 8.0 by the addition of soda lye or hydrochloric acid.

14. The method in accordance with one of claims 11 to 13,
**characterized in that** physiologically compatible gas is added to the preparation at a volume ratio of preparation to gas of 1 to 0.1 to 1 to 0.01, and small gas bubbles are created by agitation (mechanical mixing).

15. The method in accordance with claim 14,
**characterized in that** small gas bubbles are created, at least 90% of which have an average diameter of less than 3 µm.

16. The method in accordance with one of claims 11 to 15, **characterized in that** a preparation of
0.5 to 15 parts by weight of polyoxyethylene-660-12-hydroxystereate and
1 to 5 parts by weight of phospholipids and
100 parts by weight of distilled water and
0.5 to 2 parts by weight of an osmotically active substance for adjusting the pH value, such as sodium chloride or glycin,
is prepared and
100 parts by volume of the preparation are foamed up with 1 to 10 parts by volume of a physiologically compatible gas, and small gas bubbles are formed.

## Revendications

1. Produit de contraste-IV, contenant une préparation à base d'une solution aqueuse de polyoxyéthylène-660-12-hydroxystéarate et d'un phospholipide anionique.

2. Produit de contraste-IV selon la revendication 1
**caractérisé en ce que** la préparation à base d'une solution aqueuse de polyoxyéthylène-660-12-hydroxystéarate et d'un phospholipide anionique contient des gaz physiologiquement compatibles incorporés par agitation en forme de petites bulles d'air très finement réparties.

3. Produit de contraste-IV selon la revendication 1 ou 2,
**caractérisé en ce que** de l'air est utilisé comme gaz physiologiquement compatible.

4. Produit de contraste-IV selon la revendication 1 ou 2,
**caractérisé en ce que** des fluorocarbones gazeux provenant principalement du groupe C₃F₈ jusqu'à C₆F₁₄ sont utilisés comme gaz physiologiquement compatible.

5. Produit de contraste-IV selon l'une quelconque des revendications 1 ou 2,
**caractérisé en ce que** des fluorocarbones gazeux partiellement bromés sont utilisés comme gaz physiologiquement compatible.

6. Produit de contraste-IV selon l'une quelconque des revendications 1 à 5,
**caractérisé en ce que** 90 % des petites bulles de gaz présentent un diamètre moyen inférieur à 3 µm.

7. Produit de contraste-IV selon l'une quelconque des revendications 1 à 6,
**caractérisé en ce qu'**une substance active au plan osmotique, comme du chlorure de sodium, de la glycine, est contenant pour la préparation de l'isotonie physiologique.

8. Produit de contraste-IV selon l'une quelconque des revendications 1 à 7,
**caractérisé en ce que** la solution aqueuse contient
0,5 jusqu'à 15 % en poids, de préférence 1 jusqu'à 5 % en poids, de polyoxyéthylène-660-12-hydroxystéarate et
0,1 jusqu'à 10 % en poids, de préférence 0,5 jusqu'à 2 % en poids, de phospholipide.

9. Produit de contraste-IV selon l'une quelconque des revendications 1 à 8,
**caractérisé en ce que** du dimyristoylphosphatidylglycérol est utilisé comme phospholipide.

10. Produit de contraste-IV selon l'une quelconque des revendications 1 à 8,
**caractérisé en ce que** du dipalmitoylphosphatidylglycérol est utilisé comme phospholipide.

11. Procédé pour la fabrication d'un produit de contraste-IV pour une utilisation comme produit de diagnostic avec des procédés de production d'images selon l'une quelconque des revendications 1 à 10, **caractérisé en ce qu'**une préparation est fabriquée en fondant du polyoxyéthylène-660-12-hydroxystéarate à une température d'environ 65 à 75 °C, un phospholipide anionique est dissous ensuite dans cette masse fondue et ensuite de l'eau distillée et éventuellement du chlorure de sodium ou de la glycine est ajouté en remuant.

12. Procédé selon la revendication 11,
**caractérisé en ce que** l'eau distillée est ajoutée dans un état préchauffé à une température de 65 à 75 °C.

13. Procédé selon la revendication 11 ou 12,
**caractérisé en ce que** le pH de la préparation est réglé sur une valeur de 7,0 jusqu'à 8,0 par addition de lessive de soude ou d'acide chlorhydrique.

14. Procédé selon l'une quelconque des revendications 11 à 13,
**caractérisé en ce que** du gaz physiologiquement compatible est ajouté à la préparation dans le rapport de volume préparation/gaz de 1/0,1 jusqu'à 1/0,01 et des petites bulles d'air sont produites par agitation (mélange mécanique).

15. Procédé selon la revendication 14,
**caractérisé en ce que** des petites bulles d'air sont générées,
dont au moins 90 % présentent un diamètre moyen inférieur à 3 µm.

16. Procédé selon l'une quelconque des revendications 11 à 15,
**caractérisé en ce qu'**une préparation est fabriquée à partir de
0,5 jusqu'à 15 parties en poids de polyoxyéthylène-660-12-hydroxystéarate et
1 jusqu'à 5 parties en poids d'un phospholipide et 100 parties en poids d'eau distillée et
0,5 jusqu'à 2 parties ens poids d'une substance active au plan osmotique pour l'ajustage du pH, comme du chlorure de sodium ou de la glycine et
100 parties en volume de la préparation avec 1 jusqu'à 10 parties en volume d'un gaz physiologiquement compatible sont moussées en formant des petites bulles d'air.
